(19) Europäisches Patentamt
European Patent Office
Office européen des brevets

(11) Numéro de publication : **0 308 339 B1**

# (12) FASCICULE DE BREVET EUROPEEN

(45) Date de publication du fascicule du brevet : **06.05.92 Bulletin 92/19**

(51) Int. Cl.$^5$ : **C07D 209/42,** C07K 5/06

(21) Numéro de dépôt : **88402337.5**

(22) Date de dépôt : **16.09.88**

## (54) Procédé de synthèse industrielle de l'acide perhydroindole carboxylique - 2 (2S, 3aS, 7aS). Application à la synthèse decarboxyalkyl dipeptides.

(30) Priorité : **17.09.87 FR 8712900**

(43) Date de publication de la demande : **22.03.89 Bulletin 89/12**

(45) Mention de la délivrance du brevet : **06.05.92 Bulletin 92/19**

(84) Etats contractants désignés : **AT BE CH DE ES FR GB GR IT LI LU NL SE**

(56) Documents cités :
**EP-A- 0 031 741**
**EP-A- 0 037 231**
**EP-A- 0 046 953**
**EP-A- 0 049 658**
**EP-A- 0 079 022**
**EP-A- 0 093 084**
**EP-A- 0 115 345**

(56) Documents cités :
**EP-A- 0 132 580**
**EP-A- 0 154 886**
**THE CHEMISTRY OF INDOLES, Academic Press 1970, pages 129-132**

(73) Titulaire : **ADIR ET COMPAGNIE**
**1 rue Carle Hébert**
**F-92415 Courbevoie Cédex (FR)**

(72) Inventeur : **Vincent, Michel**
**8 allée du Prunier Hardy**
**F-92220 Bagneux (FR)**
Inventeur : **Baliarda, Jean**
**25 avenue Jeanne d'Arc**
**F-92160 Anthony (FR)**
Inventeur : **Marchand, Bernard**
**71 rue Laveau**
**F-45430 Checy (FR)**
Inventeur : **Remond, Georges**
**9 avenue des Etats-Unis**
**F-78000 Versailles (FR)**

EP 0 308 339 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne un nouveau procédé de synthèse industrielle de l'acide perhydroindole carboxylique - 2 (2S, 3aS, 7aS) et son application dans la synthèse industrielle de carboxyalkyl dipeptides.

Plus spécifiquement, la présente invention concerne un procédé de synthèse industrielle de l'acide perhydroindole carboxylique - 2 et son application dans la synthèse de carboxyalkyl dipeptides de formule (I) :

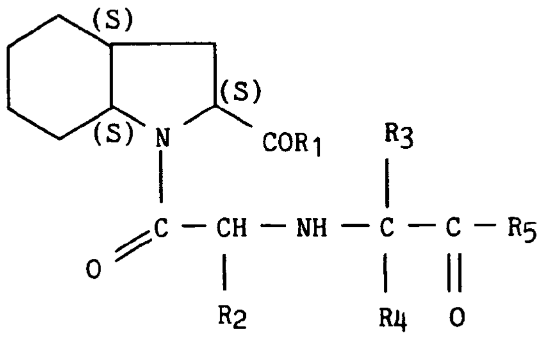

(I)

ainsi que leurs sels pharmaceutiquement acceptables,
dans laquelle :

$R_1$ et $R_5$ identiques ou différents sont hydroxy, alcoxy inférieur, alkényloxy inférieur, di-alcoylamino inférieur-alcoxy inférieur, acylamino-alcoxy inférieur, acyloxy-alcoxy inférieur, aryloxy, arylalcoxy inférieur, amino, alcoylamino inférieur, dialcoylamino inférieur, hydroxyamino, aryl-alcoylamino inférieur ou aryloxy substitué ou aryl-alcoxy inférieur substitué où le substituant est méthyle, halo ou méthoxy ;

$R_2$ est hydrogène, alcoyle inférieur, aryl-alcoyle inférieur, aminométhylphényl-alcoyle inférieur, hydroxyphényl-alcoyle inférieur, hydroxy-alcoyle inférieur, acylamino-alcoyle inférieur, amino-alcoyle inférieur, diméthylamino-alcoyle inférieur, guanidino-alcoyle inférieur, imidazolyl-alcoyle inférieur, indolyl-alcoyle inférieur ou alcoyl inférieur-thio-alcoyle inférieur ;

$R_3$ est de l'hydrogène, un alcoyle de 1 à 10 atomes de carbone linéaire ou ramifié, un alcoyle inférieur substitué par un ou plusieurs substituants choisis parmi halo, hydroxy, alcoxy inférieur, aryloxy, aryloxy substitué, hétéroaryloxy, hétéroaryloxy substitué, amino, alcoylamino inférieur, dialcoylamino inférieur, acylamino, arylamino, arylamino substitué, guanidino, imidazolyle, indolyle, alkylthio inférieur, arylthio, arylthio substitué, carboxy, carbamoyle, alcoxy inférieur-carbonyle, ou bien $R_3$ est aryle, aryle substitué, aralcoyle inférieur, aralkényl inférieur, aralcoyle inférieur substitué, aralkényl inférieur substitué, hétéroaralcoyle inférieur, hétéroaralcoyle inférieur substitué, hétéroaralkényl inférieur, hétéroaralkényl inférieur substitué, aralcoyloxy, aralcoyloxy substitué, hétéroaralcoyloxy, hétéroaralcoyloxy substitué, aralcoylthio, aralcoylthio substitué, hétéroaralcoylthio ou hétéroaralcoylthio substitué, la partie aryle ou hétéroaryle des sus-dits aryloxy, hétéroaryloxy, arylamino, arylthio, aryle, aralcoyloxy, hétéroaralcoyloxy, aralcoylthio, hétéroaralcoylthio, aralcoyle inférieur, aralkényl inférieur, hétéroaralkényl inférieur, hétéroaralcoyle inférieur substitués étant substituée par un ou plusieurs groupes choisis parmi halo, alcoyle inférieur, hydroxy, alcoxy inférieur, amino, acylamino, alcoyle inférieur amino, dialcoyle inférieur amino, carboxyle, cyano ou sulfamoyle ; la partie alcoyle des sus-dits aralcoyloxy, aralcoylthio, aralcoyle inférieur, hétéroaralcoyle inférieur, hétéroaralcoyloxy, hétéroaralcoylthio substitués étant substituée par un ou plusieurs groupes également choisis parmi halo, alcoyle inférieur, hydroxy, alcoxy inférieur, amino, acylamino, alcoyle inférieur amino, dialcoyle inférieur amino, carboxyle, cyano ou sulfamoyle ;

$R_4$ est de l'hydrogène ou un groupement alcoyle inférieur.

Parmi les valeurs ci-dessus mentionnées, il convient d'indiquer que terme "acyle" inclut les radicaux

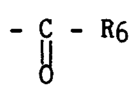

dans lesquels $R_6$ représente alcoyle inférieur, alkényle inférieur ou aryle.

Les termes alcoyle et alkényle inférieurs signifient tout radical hydrocarboné de 1 à 6 atomes de carbone, qu'ils soient linéaire ou ramifié tel que méthyle, éthyle, n - propyle, isopropyle, n - butyle, isobutyle, sec - butyle, tert.butyle, pentyle, vinyle, allyle etc...

Le terme aryle signifie, sauf indication contraire, phényle ou naphtyle éventuellement substitué par un ou

plusieurs groupement alcoyle ou alcoyloxy inférieur tel que toluyle, xylyle etc...

Le terme hétéroaryle peut être illustré par les radicaux pyridyle, thiényle, furyle, pyrrolyle, benzothiényle, benzofuryle, indolyle, thiazolyle, imidazolyle, oxazolyle, benzimidazolyle, benzothiazolyle, benzoxazolyle ainsi que l'un des précédents résultant de la substitution d'un ou plusieurs chaînons - CH - par un chaînon - N -.

Parmi les composés de formule (I) sont préférés, ceux pour lesquels :

$R_1$ et $R_5$ sont indépendamment l'un de l'autre un groupement hydroxy ou alkoxy inférieur linéaire ou ramifié,

$R_2$ est un groupement alcoyle inférieur linéaire ou ramifié éventuellement substitué par un groupement amino,

$R_3$ est un groupement alcoyle inférieur linéaire ou ramifié éventuellement substitué par un radical cycloalcoyle ou aryle tel que phényle, et parmi ceux-ci sont préférés les groupements n - propyle, n - butyle et phényl éthyle,

$R_4$ est un atome d'hydrogène.

Le composé de formule (I) préféré est le périndopril de fomule (II) :

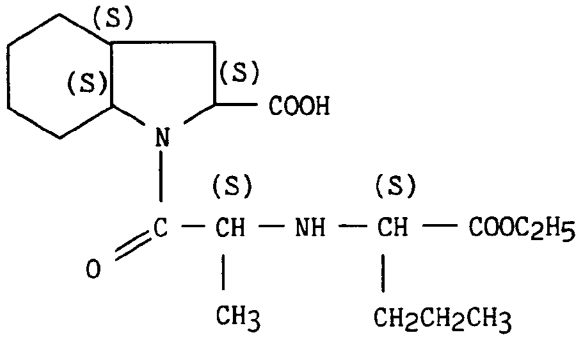

(II)

ou acide {[(éthoxycarbonyl) - 1 butylamino - (S)] - 2 propionyl - (S)} - 1 octahydroindole carboxylique - 2 (2S, 3aS, 7aS),
ainsi que ses sels d'addition à un acide ou une base pharmaceutiquement acceptables,
pour lequel le procédé de la présente invention peut être plus particulièrement appliqué.

Les composés de formule (I) ainsi que leurs sels possèdent des propriétés pharmacologiques intéressantes. Ils exercent notamment une activité inhibitrice sur certaines enzymes, comme les carboxypolypeptidases, les enképhalinases ou la kininase II. Ils inhibent notamment la transformation du décapeptide angiotensine I en l'octapeptide angiotensine II, responsable dans certains cas de l'hypertension artérielle, en agissant sur l'enzyme de conversion.

L'emploi en thérapeutique de ces composés permet donc de réduire ou même supprimer l'activité de ces enzymes responsables de la maladie hypertensive ou de l'insuffisance cardiaque. L'action sur la kininase II a pour résultat l'augmentation de la bradykinine circulante et également la baisse de la tension artérielle par cette voie.

Des composés de formule (I) leur préparation et leur utilisation en thérapeutique ont été décrits dans les brevets européens n° 0 049 658, n° 0 088 341 et dans les demandes de brevet européen n° 0 154 886, n° 0 046 953. Le dérivé de formule (II), sa préparation et son utilisation en thérapeutique ont été décrits dans le brevet européen n° 0 049 658.

En particulier, une des matières premières utilisables pour la préparation des composés de formule (I) est l'acide perhydroindole carboxylique - 2 décrit dans la demande de brevet européen n° 0 037 231, ainsi que ses esters de formule (III) :

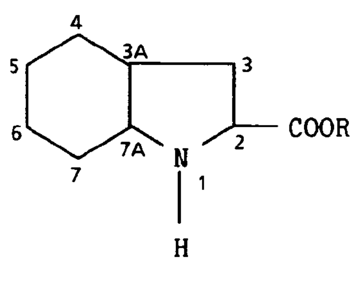

(III)

où R représente un groupement alcoyle inférieur, ou benzylique, ou un atome d'hydrogène.

Les composés de formule (III) existent sous forme de quatre paires de racémiques :

les deux épimères is IIIa et IIIb,

les deux épimères trans IIIc et IIId,

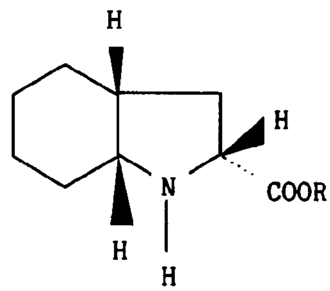

(±) IIIa
cis, endo
2S, 3aS, 7aS ou 2R, 3aR, 7aR

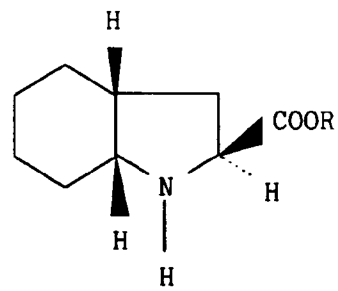

(±) IIIb
cis, exo
2S, 3aR, 7aR ou 2R, 3aS, 7aS

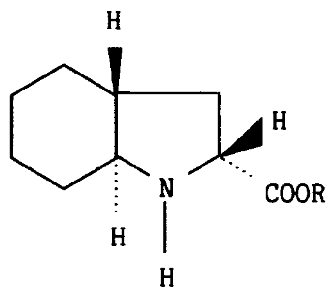

(±) IIIc
trans α
2S, 3aS, 7aR ou 2R, 3aR, 7aS

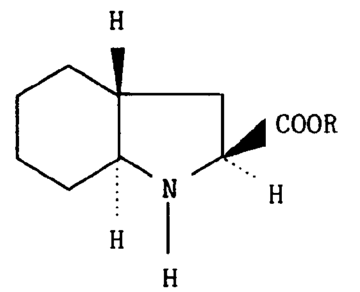

(±) IIId
trans β
2R, 3aS, 7aR ou 2S, 3aR, 7aS

La préparation de ces composés de formule (III) peut être réalisée par des techniques bien connues de l'art antérieur (EP 0 037 231, EP 0 084 164, EP 0 115 345, EP 0 173 199, EP 0 132 580).

Toutefois, l'isomère spécifiquement utilisé dans la synthèse des composés de formule (I) est l'acide perhydroindole carboxylique - 2 (2S, 3aS, 7aS) ainsi que ses esters de formule (IIIaS) :

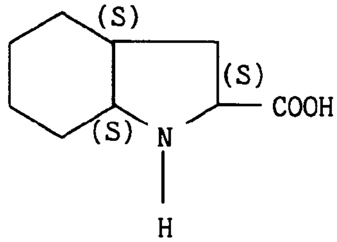

**(IIIaS)**

Il est en effet connu (EP 37231, EP 49658, EP 88341, EP 154 886), que les composés de formule (I) dont la configuration du système bicyclique est 2S, 3aS, 7aS ont une activité nettement supérieure à celle des composés pour laquelle la configuration cis du système bicyclique est différente.

La préparation de l'acide perhydroindole carboxylique - 2 (2S, 3aS, 7aS) peut être réalisée selon des techniques décrites dans l'art antérieur (EP 0 037 231, EP 0 115 345, EP 0 173 199, EP 0 132 580).

Certaines d'entre elles utilisent comme matière première l'acide indole carboxylique - 2 , qui a l'avantage d'être une matière première facilement disponible et relativement peu onéreuse (EP 0 037 231), lequel est soumis à réduction catalytique sur charbon rhodié pour donner un mélange des deux isomères cis endo de configuration respective 2S, 3aS, 7aS et 2R, 3aR, 7aR.

Cependant, la séparation de l'isomère 2S, 3aS, 7aS, utile dans la synthèse des carboxy alkyl dipeptides de formule (I), de l'isomère 2R, 3aR, 7aR nécessite généralement l'emploi de méthodes dont la mise en oeuvre est particulièrement laborieuse.

Ainsi, le brevet n° 0 037 231, pour réaliser cette séparation des isomères 2S, 3aS, 7aS et 2R, 3aR, 7aR (cis, endo racémique) utilise de nombreuses étapes nécessitant la synthèse du dérivé N - benzoylé, la cristallisation fractionnée du sel du diastéréoisomère avec la S α phényl-éthylamine, la libération des deux dérivés SSS et RRR N benzoylés, puis l'élimination du groupement benzoyle suivie d'un passage sur colonne échangeuse d'ions et d'une recristallisation.

La demande de brevet européen n° 0 115 345 pour cette même séparation, utilise plusieurs étapes nécessitant l'estérification de la fonction acide carboxylique par l'alcool benzylique, la salification de l'amino ester par la N benzyloxycarbonyl - (S) phénylalanine, la séparation par cristallisation fractionnée de l'isomère S,S,S, la libération de la fonction aminée optionnellement suivie de la libération du groupement acide carboxylique.

La demanderesse a présentement découvert un procédé original de synthèse de l'acide perhydroindole carboxylique - 2 (2S, 3aS, 7aS) présentant également l'avantage d'utiliser comme matière première l'acide indole carboxylique - 2, mais qui n'offre pas l'inconvénient de cette laborieuse séparation des deux isomères 2S, 3aS, 7aS et 2R, 3aR, 7aR de l'acide perhydroindole carboxylique, puisque l'acide indole carboxylique est, dans un premier temps réduit en acide indoline carboxylique pour donner un mélange d'acide indoline carboxylique - 2 (2R) et (2S) lesquels sont aisément séparés en une seule étape par cristallisation fractionnée ; l'isomère (2S) étant ensuite soumis à hydrogénation catalytique pour conduire stéréosélectivement, après cristallisation dans un solvant polaire rigoureusement choisi, à l'acide perhydroindole carboxylique 2 (2S, 3aS, 7aS).

Plus particulièrement, la synthèse de l'acide perhydroindole carboxylique - 2 (2S, 3aS, 7aS) découverte présentement par la demanderesse utilise comme matière première l'acide indole carboxylique - 2 ou l'un de ses esters de formule (IV) :

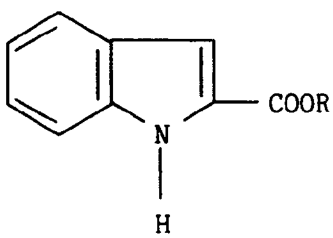

(IV)

dans lequel R représente un groupement alcoyle inférieur, ou benzyle, ou un atome d'hydrogène,
lequel est soumis à réduction par un procédé utilisant le couple étain/acide chlorhydrique à température ambiante en milieu d'alcool aliphatique inférieur, en acide indoline - 2 (R, S) ou en l'un de ses esters de formule (V) :

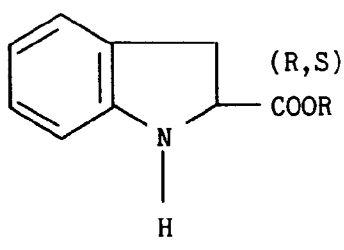

(V)

dans lequel R a la même signification que dans la formule (IV), qui, lorsque R = H, est l'acide indoline carboxylique - 2 (R, S) de formule (VI) ;
qui, lorsque R est différent de H, est transformé par hydrolyse alcaline, en acide indoline carboxylique - 2 (R, S) de formule (VI) :

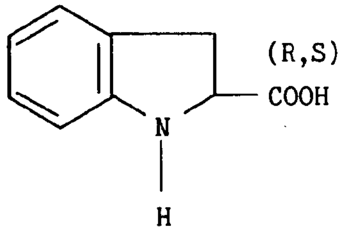

(VI)

constitué en fait, par un mélange de deux isomères selon que le carbone porteur du carboxyle se trouve :

– dans la configuration R (isomère R),

– dans la configuration S (isomère S),

mélange dont on isole l'isomère S par addition du-dit mélange à une solution de ( + ) α méthyl benzylamine dans un alcool aliphatique inférieur, pour obtenir un précipité du sel de l'acide indoline carboxylique - 2 (S) avec l' α méthyl benzylamine,

qui, après filtration, est dissous dans l'eau, la solution obtenue étant alors acidifiée pour permettre la libération de l'acide indoline carboxylique - 2 (S),

lequel est soumis à hydrogénation catalytique, le catalyseur étant choisi parmi platine, nickel, palladium, ou rhodium en mélange à un support tel que le charbon de façon à obtenir l'acide perhydroindole carboxylique - 2 (2S, 3aS, 7aS), celui ci étant séparé de l'isomère (2S, 3aR, 7aR) obtenu en faible proportion par une cristallisation unique, par un solvant rigoureusement sélectionné parmi alcool aliphatique inférieur, acétonitrile, dioxanne, acétate d'éthyle seul ou mélangés entre eux ou mélangés à l'eau à condition que le mélange soit monophasique.

Il est à remarquer que l'acide indoline carboxylique - 2 (R) peut être séparé de l'acide indoline carboxylique - 2 (R, S) en utilisant le même procédé que pour l'acide indoline - 2 (S) : il suffit alors d'utiliser la ( - ) α méthyl benzylamine.

L'invention s'étend également, aux produits originaux obtenus au cours de l'exécution de ce procédé.

L'exemple suivant illustre l'invention, mais ne la limite en aucune façon.

**EXEMPLE : ACIDE OCTAHYDROINDOLE CARBOXYLIQUE - 2 (2S, 3aS, 7aS)**

**STADE A :** Ethoxycarbonyl - 2 indole

Chauffer à ébullition 5 kg de carboxy - 2 indole, mis en suspension dans l'éthanol en présence d'acide sulfurique durant 8 heures. Evaporer l'éthanol, puis reprendre par 40 litres d'acétate d'éthyle et laver la solution organique par une solution aqueuse de soude et sécher.

Evaporer l'acétate d'éthyle, reprendre la masse cristalline par de l'hexane. Après essorage et séchage, on obtient 5,3 kg de cristaux.

Point de fusion : 123 - 125 °C

Microanalyse :

| | |
|---|---|
| Calculé | : C % 69,83 H % 5,86 N % 7,40 |
| Trouvé | : C % 69,56 H % 5,74 N % 7,30 |

Spectrométrie dans l'infra-rouge :

2150 $cm^{-1}$ (NH)

1680 $cm^{-1}$ (acide carboxylique)

**STADE B :** Ethoxycarbonyl - 2 (R, S) indoline

Dans un réacteur mettre en suspension 10 kg d'éthoxycarbonyl - 2 indoline obtenue précédemment dans 110 litres d'éthanol chlorhydrique. Ajouter ensuite, 20 kg d'étain en grenaille. Maintenir sous agitation à température ambiante pendant 2 jours environ.

Evaporer l'éthanol, reprendre le résidu par l'eau et ajouter 110 litres de toluène. Agiter 20 minutes environ.

Alcaliniser par l'ammoniaque.
Décanter la phase aqueuse et l'extraire une nouvelle fois par 150 litres de toluène.

Réunir les phases toluéniques, et les laver à l'eau. Décanter les phases toluéniques, filtrer.

Eliminer l'eau par distillation de l'azéotrope eau toluène. Refroidir et faire passer un courant d'HCl gazeux anhydre.

Refroidir. Evaporer, et laver par du toluène pur.

Poids obtenu : 10,11 kg

Rendement : 84 %

Chromatographie sur couche mince :

Solvant : toluène : 10
acétate d'éthyle : 5
Support : silice 60 F 254 MERCK
Révélateur : UV
Rf.: 0,55

**STADE C :** Carboxy - 2 (R, S) indoline

2,15 kg d'éthoxycarbonyl - 2 (R, S) indoline en solution dans l'éthanol sont saponifiés par 12,5 litres de soude N, sous agitation pendant 24 heures. Après lavage de la solution alcaline, neutraliser par l'acide chlorhydrique concentré. Après essorage, lavage, séchage, on obtient 1,57 kg de cristaux blancs du produit attendu.

Rendement : 86 %.

Point de fusion : 188 - 189 °C

Spectrométrie dans l'infra-rouge :

$NH_2^+$ : 2500 - 2000 $cm^{-1}$
$COO^-$ : 1620 $cm^{-1}$

**STADE D :** Carboxy - 2 (S) indoline

6,05 kg de (R, S) carboxy - 2 indoline sont ajoutés à une solution de 4,49 kg d'( + ) αméthyl benzylamine dans l'éthanol anhydre. On obtient un produit précipité blanc qui, après essorage est digéré dans de l'isopropanol chauffé au reflux. Après refroidissement, on essore, lave avec un peu d'isopropanol, les cristaux blancs obtenus sont séchés : 3,68 kg.

Pouvoir rotatoire :

$$[\alpha]^{D}_{21} = -5{,}3 \ (c = 1\ \%\mathit{éthanol})$$

La (S) carboxy - 2 indoline est préparée avec un rendement quantitatif par dissolution de 1 kg du sel précédent dans 5 litres d'eau et neutralisation par une solution aqueuse d'acide chlorhydrique. Ce précipité est essoré, lavé à l'eau et séché.

**STADE E :** Acide octahydroindole carboxylique - 2 (2S, 3aS, 7aS)

Dans une cuve, placer 25 kg d'acide indoline carboxylique - 2 (S), précédemment obtenus dans 110 litres de méthanol. Maintenir sous agitation. Dans un mélangeur, charger le catalyseur au rhodium (5 % sec).

Dans un hydrogénateur, mettre l'agitation en route, et charger la suspension méthanolique d'acide indoline

carboxylique - 2 (S) en la faisant transiter par le mélangeur et rincer l'ensemble à l'eau. Porter à 60 °C et mettre en pression d'hydrogène.

Filtrer le catalyseur sur filtre monoplaque. Recueillir les jus hydroalcooliques dans un réacteur, et évaporer le méthanol sous vide. Après concentration, charger 300 kg environ de dioxanne. Porter à ébullition et ajouter de l'eau jusqu'à l'obtention d'une solution. Laisser refroidir. Essorer et sécher.

On obtient 22,3 kg de cristaux.

Rendement : 86,1 %.

## Revendications

### Revendications pour les Etats contractants suivants : AT BE CH DE FR GB IT LI LU NL SE

1. Procédé de préparation industrielle de l'acide perhydroindole carboxylique - 2 (2S, 3aS, 7aS) de formule (IIIaS) :

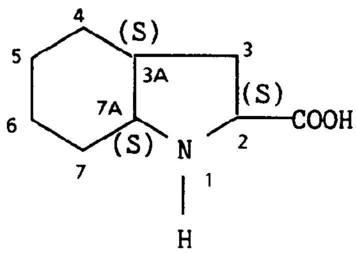

**(IIIaS)**

caractérisé en ce que l'on utilise comme matière première l'acide indole carboxylique - 2 où l'un de ses esters de formule (IV) :

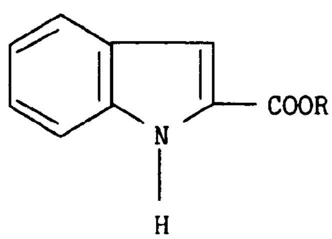

**(IV)**

éventuellement salifié par un acide AH,
dans laquelle R représente un atome d'hydrogène ou un groupement alcoyle inférieur,
lequel est soumis à réduction par un procédé utilisant le couple étain acide chlorhydrique,
pour conduire à l'acide indoline carboxylique - 2 (R, S) ou à l'un de ses esters de formule (V) :

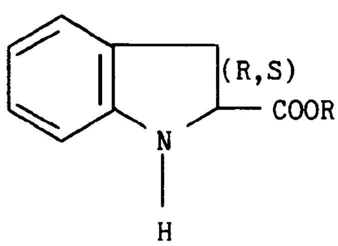

**(V)**

dans laquelle R a la même signification que dans la formule (IV), qui, lorsque R = H, est l'acide indoline carboxylique - 2 (R, S) de formule (VI) ;
qui, lorsque R est différent de H est transformé par hydrolyse alcaline en acide indoline carboxylique - 2 (R, S) de formule (VI) :

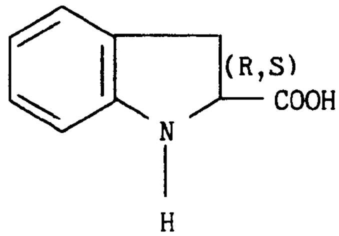

(VI)

constitué en fait, par un mélange de deux isomères selon que le carbone porteur du carboxyle se trouve :

– dans la configuration R (isomère R),

– dans la configuration S (isomère S),

mélange dont on isole l'isomère S par addition du-dit mélange à une solution de ( + ) α méthyl benzylamine dans un alcool aliphatique inférieur, pour obtenir,

un précipité du sel de l'acide indoline carboxylique - 2 (S) avec la ( + ) α méthyl benzylamine,

qui, après filtration, est dissous dans l'eau, la solution obtenue étant alors acidifiée pour permettre la libération de l'acide indoline - 2 (S) carboxylique,

lequel est soumis à hydrogénation catalytique, le catalyseur étant choisi parmi nickel, platine, palladium, rhodium en mélange à un support tel que le charbon de façon à obtenir l'acide perhydroindole carboxylique - 2 (2S, 3aS, 7aS), celui ci étant séparé de l'isomère (2S, 3aR, 7aR) obtenu en faible proportion par une cristallisation unique dans un solvant polaire soigneusement choisi parmi alcool aliphatique inférieur, acétonitrile, dioxanne, acétate d'éthyle seul ou mélangés entre eux ou mélangés à l'eau à condition que le mélange soit monophasique.

2. Procédé de synthèse industrielle de l'acide perhydroindole carboxylique - 2 (2S, 3aS, 7aS) selon la revendication 1, caractérisé en ce que l'on utilise comme matière première l'ester éthylique de l'acide indole carboxylique - 2 de formule (IVo) :

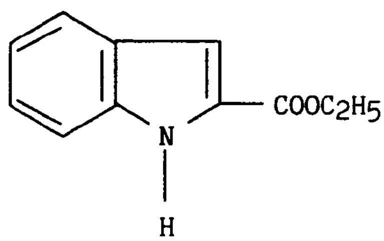

(IVo)

lui-même obtenu par estérification par l'éthanol de l'acide indole carboxylique - 2 en présence d'un catalyseur d'estérification tel que l'acide sulfurique.

3. Procédé selon l'une des revendications 1 ou 2 caractérisé en ce que la réduction de l'acide indoline carboxylique - 2 (S) en acide perhydroindole carboxylique - 2 s'effectue en utilisant comme catalyseur le rhodium sur charbon.

4. Procédé selon l'une quelconque des revendications 1 à 3 caractérisé en ce que la recristallisation ayant pour but d'isoler l'acide perhydroindole carboxylique - 2 (2S, 3aS, 7aS) à partir du mélange réactionnel obtenu après réduction catalytique de l'acide indoline carboxylique - 2 (S) s'effectue en utilisant comme solvant le mélange dioxanne - eau.

5. Utilisation de l'acide perhydroindole carboxylique - 2 (2S, 3aS, 7aS) obtenu selon l'une quelconque des revendications 1 à 4 pour la préparation de carboxyalkyl dipeptides de formule (I) :

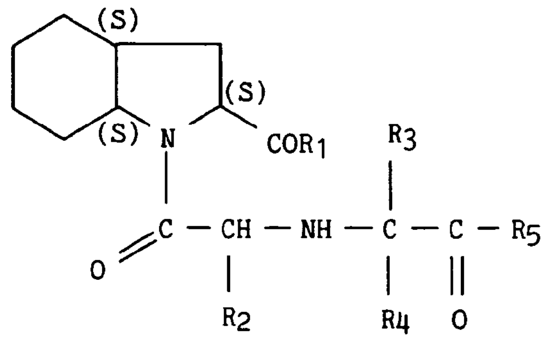

(I)

ainsi que leurs sels pharmaceutiquement acceptables, dans laquelle :

$R_1$ et $R_5$ identiques ou différents sont hydroxy, alcoxy inférieur, alkényloxy inférieur, di-alcoylamino inférieur-alcoxy inférieur, acylamino-alcoxy inférieur, acyloxy-alcoxy inférieur, aryloxy, arylalcoxy inférieur, amino, alcoylamino inférieur, dialcoylamino inférieur, hydroxyamino, aryl-alcoylamino inférieur ou aryloxy substitué ou aryl-alcoxy inférieur substitué où le substituant est méthyle, halo ou méthoxy ;

$R_2$ est hydrogène, alcoyle inférieur, aryl-alcoyle inférieur, aminomethylphényl-alcoyle inférieur, hydroxyphényl-alcoyle inférieur, hydroxy-alcoyle inférieur, acylamino-alcoyle inférieur, amino-alcoyle inférieur, diméthylamino-alcoyle inférieur, guanidino-alcoyle inférieur, imidazolyl-alcoyle inférieur, indolyl-alcoyle inférieur ou alcoyl inférieur-thio-alcoyle inférieur ;

$R_3$ est de l'hydrogène, un alcoyle de 1 à 10 atomes de carbone linéaire ou ramifié, un alcoyle inférieur substitué par un ou plusieurs substituants choisis parmi halo, hydroxy, alcoxy inférieur, aryloxy, aryloxy substitué, hétéroaryloxy, hétéroaryloxy substitué, amino, alcoylamino inférieur, dialcoylamino inférieur, acylamino, arylamino, arylamino substitué, guanidino, imidazolyle, indolyle, alkylthio inférieur, arylthio, arylthio substitué, carboxy, carbamoyle, alcoxy inférieur-carbonyle, ou bien $R_3$ est aryle, aryle substitué, aralcoyle inférieur, aralkényl inférieur, aralcoyle inférieur substitué, aralkényl inférieur substitué, hétéroaralcoyle inférieur, hétéroaralcoyle inférieur substitué, hétéroaralkényl inférieur, hétéroaralkényl inférieur substitué, aralcoyloxy, aralcoyloxy substitué, hétéroaralcoyloxy, hétéroaralcoyloxy substitué, aralcoylthio, aralcoylthio substitué, hétéroaralcoylthio ou hétéroaralcoylthio substitué, la partie aryle ou hétéroaryle des sus-dits aryloxy, hétéroaryloxy, arylamino, arylthio, aryle, aralcoyloxy, hétéroaralcoyloxy, aralcoylthio, hétéroaralcoylthio, aralcoyle inférieur, aralkényl inférieur, hétéroaralkényl inférieur, hétéroaralcoyle inférieur substitués étant substituée par un ou plusieurs groupes choisis parmi halo, alcoyle inférieur, hydroxy, alcoxy inférieur, amino, acylamino, alcoyle inférieur amino, dialcoyle inférieur amino, carboxyle, cyano ou sulfamoyle ; la partie alcoyle des sus-dits aralcoyloxy, aralcoylthio, aralcoyle inférieur, hétéroaralcoyle inférieur, hétéroaralcoyloxy, hétéroaralcoylthio substitués étant substituée par un ou plusieurs groupes également choisis parmi halo, alcoyle inférieur, hydroxy, alcoxy inférieur, amino, acylamino, alcoyle inférieur amino, dialcoyle inférieur amino, carboxyle, cyano ou sulfamoyle ;

$R_4$ est de l'hydrogène ou un groupement alcoyle inférieur.

6. Utilisation selon la revendication 5 de l'acide perhydroindole carboxylique - 2 (2S, 3aS, 7aS) pour la préparation industrielle de l'acide {[(éthoxycarbonyl) - 1 butylamino (S)] - 2 propionyl - (S)} - 1 octahydroindole carboxylique - 2 - (2S, 3aS, 7aS) de son sel de tert.butylamine.

**Revendications pour les Etats contractants suivants : ES GR**

1. Procédé de préparation industrielle de l'acide perhydroindole carboxylique - 2 (2S, 3aS, 7aS) de formule (IIIaS) :

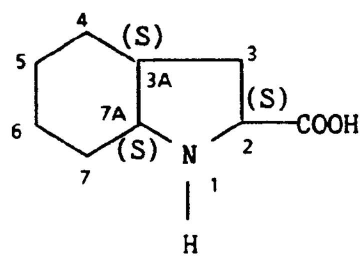

**(IIIaS)**

caractérisé en ce que l'on utilise comme matière première l'acide indole carboxylique - 2 où l'un de ses esters de formule (IV) :

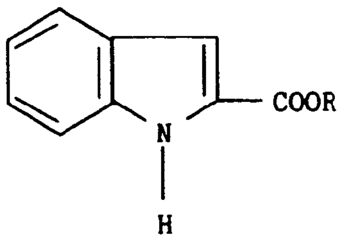

**(IV)**

éventuellement salifié par un acide AH,
dans laquelle R représente un atome d'hydrogène ou un groupement alcoyle inférieur,
lequel est soumis à réduction par un procédé utilisant le couple étain acide chlorhydrique,
pour conduire à l'acide indoline carboxylique - 2 (R, S) ou à l'un de ses esters de formule (V) :

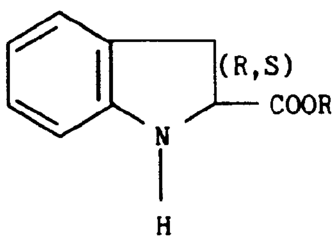

**(V)**

dans laquelle R a la même signification que dans la formule (IV), qui, lorsque R = H, est l'acide indoline carboxylique - 2 (R, S) de formule (VI) ;
qui, lorsque R est différent de H est transformé par hydrolyse alcaline en acide indoline carboxylique - 2 (R, S) de formule (VI) :

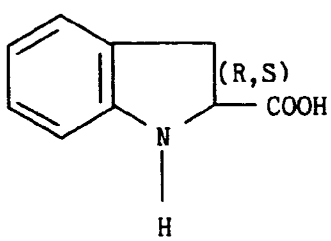

**(VI)**

constitué en fait, par un mélange de deux isomères selon que le carbone porteur du carboxyle se trouve :
- dans la configuration R (isomère R),
- dans la configuration S (isomère S),

mélange dont on isole l'isomère S par addition du-dit mélange à une solution de ( + ) αmethyl benzylamine dans un alcool aliphatique inférieur, pour obtenir,
un précipité du sel de l'acide indoline carboxylique - 2 (S) avec la ( + )αméthyl benzylamine,
qui, après filtration, est dissous dans l'eau, la solution obtenue étant alors acidifiée pour permettre la libération de l'acide indoline - 2 (S) carboxylique,
lequel est soumis à hydrogénation catalytique, le catalyseur étant choisi parmi nickel, platine, palladium, rhodium en mélange à un support tel que le charbon de façon à obtenir l'acide perhydroindole carboxylique - 2 (2S, 3aS, 7aS), celui ci étant séparé de l'isomère (2S, 3aR, 7aR) obtenu en faible proportion par une cristallisation unique dans un solvant polaire soigneusement choisi parmi alcool aliphatique inférieur, acétonitrile, dioxanne, acétate d'éthyle seul ou mélangés entre eux ou mélanges à l'eau à condition que le mélange soit monophasique.

2. Procédé de synthèse industrielle de l'acide perhydroindole carboxylique - 2 (2S, 3aS, 7aS) selon la revendication 1, caractérisé en ce que l'on utilise comme matière première l'ester éthylique de l'acide indole carboxylique - 2 de formule (IVo) :

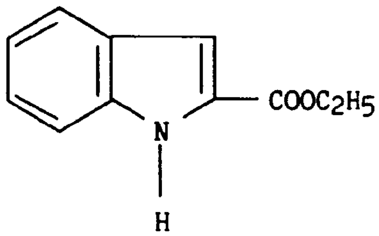

(IVo)

lui-même obtenu par estérification par l'éthanol de l'acide indole carboxylique - 2 en présence d'un catalyseur d'estérification tel que l'acide sulfurique.

3. Procédé selon l'une des revendications 1 ou 2 caractérisé en ce que la réduction de l'acide indoline carboxylique - 2 (S) en acide perhydroindole carboxylique - 2 s'effectue en utilisant comme catalyseur le rhodium sur charbon.

4. Procédé selon l'une quelconque des revendications 1 à 3 caractérisé en ce que la recristallisation ayant pour but d'isoler l'acide perhydroindole carboxylique - 2 (2S, 3aS, 7aS) à partir du mélange réactionnel obtenu après réduction catalytique de l'acide indoline carboxylique - 2 (S) s'effectue en utilisant comme solvant le mélange dioxanne - eau.

5. Utilisation de l'acide perhydroindole carboxylique - 2 (2S, 3aS, 7aS) obtenu selon l'une quelconque des revendications 1 à 4 pour la préparation de carboxyalkyl dipeptides de formule (I) :

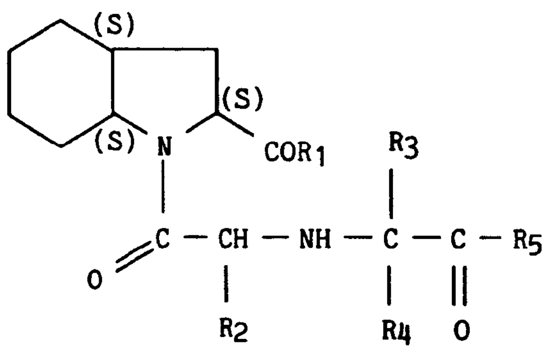

(I)

ainsi que leurs sels pharmaceutiquement acceptables, dans laquelle :

$R_1$ et $R_5$ identiques ou différents sont hydroxy, alcoxy inférieur, alkényloxy inférieur, di-alcoylamino inférieur-alcoxy inférieur, acylamino-alcoxy inférieur, acyloxy-alcoxy inférieur, aryloxy, aryl-alcoxy inférieur, amino, alcoylamino inférieur, dialcoylamino inférieur, hydroxyamino, aryl-alcoylamino inférieur ou aryloxy substitué ou aryl-alcoxy inférieur substitué où le substituant est méthyle, halo ou méthoxy ;

$R_2$ est hydrogène, alcoyle inférieur, aryl-alcoyle inférieur, aminométhylphényl-alcoyle inférieur, hydroxyphényl-alcoyle inférieur, hydroxy-alcoyle inférieur, acylamino-alcoyle inférieur, amino-alcoyle inférieur, diméthylamino-alcoyle inférieur, guanidino-alcoyle inférieur, imidazolyl-alcoyle inférieur, indolyl-alcoyle inférieur ou alcoyl inférieur-thio-alcoyle inférieur ;

$R_3$ est de l'hydrogène, un alcoyle de 1 à 10 atomes de carbone linéaire ou ramifié, un alcoyle inférieur substitué par un ou plusieurs substituants choisis parmi halo, hydroxy, alcoxy inférieur, aryloxy, aryloxy substitué, hétéroaryloxy, hétéroaryloxy substitué, amino, alcoylamino inférieur, dialcoylamino inférieur, acylamino, arylamino, arylamino substitué, guanidino, imidazolyle, indolyle, alkylthio inférieur, arylthio, arylthio substitué, carboxy, carbamoyle, alcoxy inférieur-carbonyle, ou bien $R_3$ est aryle, aryle substitué, aralcoyle inférieur, aralkenyl inférieur, aralcoyle inférieur substitué, aralkényl inférieur substitué, héteroaralcoyle inférieur, héteéoaralcoyle inférieur substitué, hétéroaralkenyl inférieur, hétéroaralkenyl inférieur substitué, aralcoyloxy, aralcoyloxy substitué, hétéroaralcoyloxy, hétéroaralcoyloxy substitué, aralcoylthio, aralcoylthio substitué, hétéroaralcoylthio ou hétéroaralcoylthio substitué, la partie aryle ou hétéroaryle des sus-dits aryloxy, hétéroaryloxy, arylamino, arylthio, aryle, aralcoyloxy, hétéroaralcoyloxy, aralcoylthio, hétéroaralcoylthio, aralcoyle inférieur, aralkényl inférieur, hétéroaralkényl inférieur, hétéroaralcoyle inférieur substitués étant substituée par un ou plusieurs groupes choisis parmi halo, alcoyle inférieur, hydroxy, alcoxy inférieur, amino, acylamino, alcoyle inférieur amino, dialcoyle inférieur amino, carboxyle, cyano ou sulfamoyle ; la partie alcoyle des sus-dits aralcoyloxy, aralcoylthio, aralcoyle inférieur, hétéroaralcoyle inférieur, hétéroaralcoyloxy, hétéroaralcoylthio substitues étant substituée par un ou plusieurs groupes également choisis parmi halo, alcoyle inférieur, hydroxy, alcoxy inférieur, amino, acylamino, alcoyle inférieur amino, dialcoyle inférieur amino, carboxyle, cyano

ou sulfamoyle ;

$R_4$ est de l'hydrogène ou un groupement alcoyle inférieur.

6. Utilisation selon la revendication 5 de l'acide perhydroindole carboxylique - 2 (2S, 3aS, 7aS) pour la préparation industrielle de l'acide {((éthoxycarbonyl) - 1 butylamino (S)] - 2 propionyl - (S)} - 1 octahydroindole carboxylique - 2 - (2S, 3aS, 7aS) de son sel de tert. butylamine.

## Patentansprüche

### Patentansprüche für folgende Vertragsstaaten : AT BE CH DE FR GB IT LI LU NL SE

1. Verfahren zur technischen Herstellung von (2S, 3aS, 7aS)-Perhydroindo1-2-carbonsäure der Formel (IIIaS):

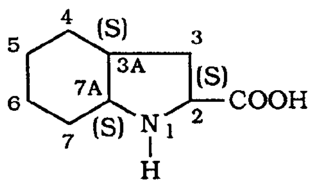

(IIIaS)

**dadurch gekennzeichnet**, daß man als Ausgangsmaterial Indol-2-carbonsäure oder einen ihrer Ester der Formel (IV):

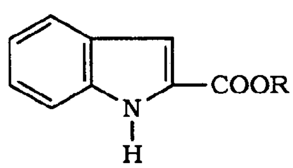

(IV)

gegebenenfalls in Form eines Salzes mit einer Säure AH,
in der R ein Wasserstoffatom oder eine niedrigmolekulare Alkylgruppe bedeutet,
verwendet, welche man einer Reduktion unter Verwendung der Kombination Zinn/Chlorwasserstoffsäure unterwirft zur Bildung der Indolin-2(R, S)-carbonsäure oder eines ihrer Ester der Formel (V):

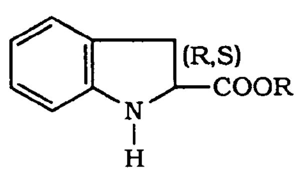

(V)

in der R die bezüglich der Formel (IV) angegebenen Bedeutungen besitzt, welche, wenn R = H, die Indolin-2(R, S)-carbonsäure der Formel (VI) ist;
welchen man dann, wenn R von Wasserstoff verschieden ist, durch alkalische Hydrolyse in Indolin-2(R, S)-carbonsäure der Formel (VI):

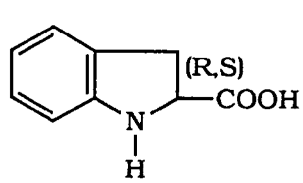

(VI)

umwandelt, welche in Form einer Mischung der beiden Isomeren bezüglich des die Carboxylgruppe tragenden

Kohlenstoffatoms vorliegt, nämlich:
– in der Konfiguration R (Isomeres R),
– in der Konfiguration S (Isomeres S),
aus welcher Mischung man das Isomere S isoliert durch Zugabe einer Lösung von (+)-α-Methyl-benzylamin in einem niedrigmolekularen Alkohol zu der Mischung zur Bildung eines Niederschlags des Salzes aus der Indolin-2(S)-carbonsäure mit (+)-α-Methyl-benzylamin, welche man nach dem Abfiltrieren in Wasser löst und die erhaltene Lösung ansäuert zur Freisetzung der Indolin-2(S)-carbonsäure,
welche man einer katalytischen Hydrierung mit einem Katalysator ausgewählt aus Nickel, Platin, Palladium oder Rhodium in Mischung mit einem Trägermaterial, wie Kohlenstoff, unterwirft zur Bildung der (2S, 3aS, 7aS)-Perhydroindol-2-carbonsäure, welche von dem (2S, 3aR, 7aR)-Isomeren, welches in geringen Mengen anfällt, abtrennt durch einmalige Kristallisation in einem polaren Lösungsmittel, welches sorgfältig ausgewählt ist aus niedrigmolekularen aliphatischen Alkoholen, Acetonitril, Dioxan, Ethylacetat als solche oder in Form von Mischungen untereinander oder in Form von Mischungen mit Wasser mit der Maßgabe, daß die Mischung einphasig ist.

2. Verfahren zur technischen Herstellung von (2S, 3aS, 7aS)-Perhydroindol-2-carbonsäure nach Anspruch 1, **dadurch gekennzeichnet**, daß man als Ausgangsmaterial den Ethylester der Indol-2-carbonsäure der Formel (IVo):

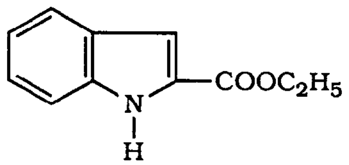

(VIo)

einsetzt, der seinerseits durch Verestern der Indol-2-carbonsäure mit Ethanol in Gegenwart eines Veresterungskatalysators, wie Schwefelsäure, hergestellt worden ist.

3. Verfahren nach einem der Ansprüche 1 oder 2, **dadurch gekennzeichnet,** daß die Reduktion der Indolin-2(S)-carbonsäure zu der Perhydroindol-2-carbonsäure unter Anwendung von Rhodium-auf-Kohlenstoff als Katalysator erfolgt.

4. Verfahren nach einem der Ansprüche 1 bis 3, **dadurch gekennzeichnet**, daß die Umkristallisation zur Isolierung der (2S, 3aS, 7aS)-Perhydroindol-2-carbonsäure ausgehend von der nach der katalytischen Reduktion der Indolin-2(S)-carbonsäure erhaltenen Mischung unter Verwendung einer Dioxan/Wasser-Mischung als Lösungsmittel durchgeführt wird.

5. Verwendung der (2S, 3aS, 7aS)-Perhydroindol-2-carbonsäure erhalten nach einem der Ansprüche 1 bis 4 zur Herstellung von Carboxalkyldipeptiden der Formel (I):

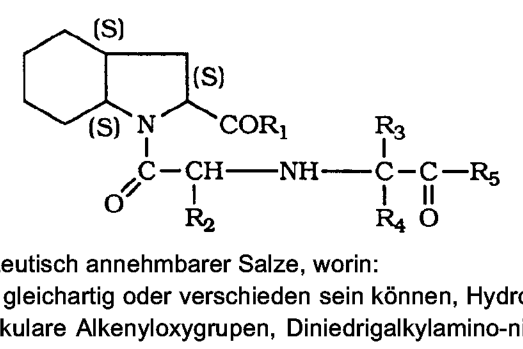

(I)

sowie deren pharmazeutisch annehmbarer Salze, worin:

$R_1$ und $R_5$, die gleichartig oder verschieden sein können, Hydroxylgruppen, niedrigmolekulare Alkoxygruppen, niedrigmolekulare Alkenyloxygrupen, Diniedrigalkylamino-niedrigalkoxygruppen, Acylamino-niedrigalkoxygruppen, Acyloxy-niedrigalkoxygruppen, Aryloxygruppen, Aryl-niedrigalkoxygruppen, Aminogruppen, Niedrigalkylaminogruppen, Diniedrigalkylaminogruppen, Hydroxyaminogruppen, Aryl-niedrigalkylaminogruppen oder substituierte Aryloxygruppen oder substituierte Aryl-niedrigalkoxygruppen, worin der Substituent eine Methylgruppe, ein Halogenatom oder eine Methoxygruppe ist;

$R_2$ Wasserstoff, eine Niedrigalkylgruppe, eine Aryl-niedrigalkylgruppe, eine Aminomethylphenyl-niedrigalkylgruppe, eine Hydroxyphenyl-niedrigalkylgruppe, eine Hydroxy-niedrigalkylgruppe, eine Acylamino-niedrigalkylgruppe, eine Amino-niedrigalkylgruppe, eine Dimethylamino-niedrigalkylgruppe, eine Guanidino-niedrigalkylgruppe, eine Imidazolyl-niedrigalkylgruppe, eine Indolyl-niedrigalkylgruppe oder eine Niedrigalkyl-niedrigthioalkylgruppe;

$R_3$ ein Wasserstoffatom, eine geradkettige oder verzweigte Alkylgruppe mit 1 bis 10 Kohlenstoffatomen, eine Niedrigalkylgruppe, substituiert mit einem oder mehreren Substituenten, ausgewählt aus Halogenatomen, Hydroxylgruppen, Niedrigalkoxygruppen, Aryloxygruppen, substituierten Aryloxygruppen, Heteroaryloxygruppen, substituierten Heteroaryloxygruppen, Aminogruppen, Niedrigalkylaminogruppen, Diniedrigalkylaminogruppen, Acylaminogruppen, Arylaminogruppen, substituierten Arylaminogruppen, Guanidinogruppen, Imidazolylgruppen, Indolylgruppen, Niedrigalkylthiogruppen, Arylthiogruppen, substituierten Arylthiogruppen, Carboxygruppen, Carbamoylgruppen oder Niedrigalkoxycarbonylgruppen, oder $R_3$ eine Arylgruppe, eine substituierte Arylgruppe, eine Niedrigaralkylgruppe, eine Niedrigaralkenylgruppe, eine substituierte Niedrigaralkylgruppe, eine substituierte Niedrigaralkenylgruppe, eine Niedrigheteroaralkylgruppe, eine substituierte Niedrigheteroaralkylgruppe, eine Niedrigheteroaralkenylgruppe, eine substituierte Niedrigheteroaralkenylgruppe, eine Aralkyloxygruppe, eine substituierte Aralkyloxygruppe, eine Heteroaralkyloxygruppe, eine substituierte Heteroaralkyloxygruppe, eine Aralkylthiogruppe, eine substituierte Aralkylthiogruppe, eine Heteroaralkylthiogruppe oder eine substituierte Heteroaralkylthiogruppe, wobei der Arylrest oder der Heteroarylrest der genannten Aryloxygruppen, Heteroaryloxygruppen, Arylaminogruppen, Arylthiogruppen, Arylgruppen, Aralkyloxygruppen, Heteroaralkyloxygruppen, Aralkylthiogruppen, Heteroaralkylthiogruppen, Niedrigaralkylgruppen, Niedrigaralkenylgruppen, Niedrigheteroarlkenylgruppen, substituierten Niedrigheteroaralkylgruppen durch eine oder mehrere Gruppen ausgewählt aus Halogenatomen, Niedrigalkylgruppen, Hydroxygruppen, Niedrigalkoxygruppen, Aminogruppen, Acylaminogruppen, Niedrigalkylaminogruppen, Diniedrigalkylaminogruppen, Carboxylgruppen, Cyanogruppen oder Sulfamoylgruppen substituiert sind; der Alkylrest der genannten Aralkyloxygruppen, Aralkylthiogruppen, Niedngaralkylgruppen, Niedrigheteroaralkylgruppen, Heteroaralkyloxygruppen und substituierten Heteroalkylthiogruppen durch eine oder mehrere Gruppen substituiert ist ausgewählt aus Halogenatomen, Niedrigalkylgruppen, Hydroxygruppen, Niedrigalkoxygruppen, Aminogruppen, Acylaminogruppen, Niedrigalkylaminogruppen, Diniedrigalkylaminogruppen, Carboxylgruppen, Cyanogruppen oder Sulfamoylgruppen substituiert sind; und

$R_4$ ein Wasserstoffatom oder eine Niedrigalkylgruppe bedeuten.

6. Verwendung der (2S, 3aS, 7aS)-Perhydroindol-2-carbonsäure nach Anspruch 5 zur technischen Herstellung von (2S, 3aS, 7aS)-1-{2-[1-(Ethoxycarbonyl)-(S)-butylamino]-(S)-propionyl}-octahydroindol-2-carbonsäure und deren tert.-Butylaminsalz.

**Patentansprüche für folgende Vertragsstaaten : ES, GR**

1. Verfahren zur technischen Herstellung von (2S, 3aS, 7aS)-Perhydroindol-2-carbonsäure der Formel (IIIaS):

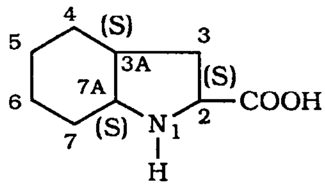

(IIIaS)

**dadurch gekennzeichnet**, daß man als Ausgangsmaterial Indol-2-carbonsäure oder einen ihrer Ester der Formel (IV):

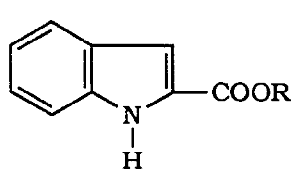

(IV)

gegebenenfalls in Form eines Salzes mit einer Säure AH,
in der R ein Wasserstoffatom oder eine niedrigmolekulare Alkylgruppe bedeutet,
verwendet, welche man einer Reduktion unter Verwendung der Kombination Zinn/Chlorwasserstoffsäure unterwirft zur Bildung der Indolin-2(R,S)-carbonsäure oder eines ihrer Ester der Formel (V):

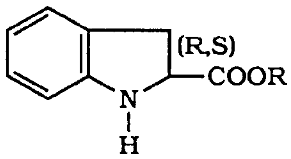

(V)

in der R die bezüglich der Formel (IV) angegebenen Bedeutungen besitzt, welche, wenn R = H, die Indolin-2(R,S)-carbonsäure der Formel (VI) ist;
welchen man dann, wenn R von Wasserstoff verschieden ist, durch alkalische Hydrolyse in Indolin-2(R,S)-carbonsäure der Formel (VI):

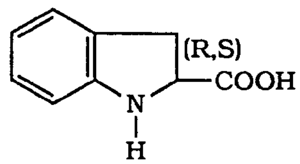

(VI)

umwandelt, welche in Form einer Mischung der beiden Isomeren bezüglich des die Carboxylgruppe tragenden Kohlenstoffatoms vorliegt, nämlich:

- in der Konfiguration R (Isomeres R),
- in der Konfiguration S (Isomeres S),

aus welcher Mischung man das Isomere S isoliert durch Zugabe einer Lösung von (+)-α-Methyl-benzylamin in einem niedrigmolekularen Alkohol zu der Mischung zur Bildung eines Niederschlags des Salzes aus der Indolin-2(S)-carbonsäure mit (+)-α-Methyl-benzylamin, welche man nach dem Abfiltrieren in Wasser löst und die erhaltene Lösung ansäuert zur Freisetzung der Indolin-2(S)-carbonsäure,
welche man einer katalytischen Hydrierung mit einem Katalysator ausgewählt aus Nickel, Platin, Palladium oder Rhodium in Mischung mit einem Trägermaterial, wie Kohlenstoff, unterwirft zur Bildung der (2S, 3aS, 7aS)-Perhydroindol-2-carbonsäure, welche von dem (2S, 3aR, 7aR)-Isomeren, welches in geringen Mengen anfällt, abtrennt durch einmalige Kristallisation in einem polaren Lösungsmittel, welches sorgfältig ausgewählt ist aus niedrigmolekularen aliphatischen Alkoholen, Acetonitril, Dioxan, Ethylacetat als solche oder in Form von Mischungen untereinander oder in Form von Mischungen mit Wasser mit der Maßgabe, daß die Mischung einphasig ist.

2. Verfahren zur technischen Herstellung von (2S, 3aS, 7aS)-Perhydroindol-2-carbonsäure nach Anspruch 1, **dadurch gekennzeichnet**, daß man als Ausgangsmaterial den Ethylester der Indol-2-carbonsäure der Formel (IVo):

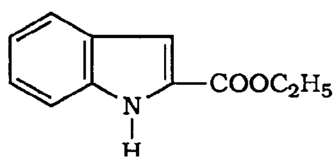

(VIo)

einsetzt, der seinerseits durch Verestern der Indol-2-carbonsäure mit Ethanol in Gegenwart eines Veresterungskatalysators, wie Schwefelsäure, hergestellt worden ist.

3. Verfahren nach einem der Ansprüche 1 oder 2, **dadurch gekennzeichnet**, daß die Reduktion der Indolin-2(S)-carbonsäure zu der Perhydroindol-2-carbonsäure unter Anwendung von Rhodium-auf-Kohlenstoff als Katalysator erfolgt.

4. Verfahren nach einem Ansprüche 1 bis 3, **dadurch gekennzeichnet**, daß die Umkristallisation zur Isolierung der (2S, 3aS, 7aS)-Perhydroindol-2-carbonsäure ausgehend von der nach der katalytischen Reduktion der Indolin-2(S)-carbonsäure erhaltenen Mischung unter Verwendung einer Dioxan/Wasser-Mischung als Lösungsmittel durchgeführt wird.

5. Verwendung der (2S, 3aS, 7aS)-Perhydroindol-2-carbonsäure erhalten nach einem der Ansprüche 1 bis 4 zur Herstellung von Carboxyalkyldipeptiden der Formel (I):

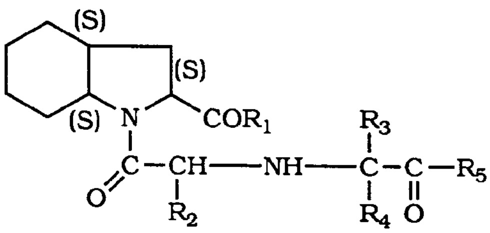

(I)

sowie deren pharmazeutisch annehmbarer Salze, worin:

$R_1$ und $R_5$, die gleichartig oder verschieden sein können, Hydroxylgruppenn niedrigmolekulare Alkoxygruppen, niedrigmolekulare Alkenyloxygrupen, Diniedrigalkylamino-niedrigalkoxygruppen, Acylamino-niedrigalkoxygruppen, Acyloxy-niedrigalkoxygruppen, Aryloxygruppen, Aryl-niedrigalkoxygruppen, Aminogruppen, Niedrigalkylaminogruppen, Diniedrigalkylaminogruppen, Hydroxyaminogruppen, Aryl-niedrigalkylaminogruppen oder substituierte Aryloxygruppen oder substituierte Aryl-niedrigalkoxygruppen, worin der Substituent eine Methylgruppe, ein Halogenatom oder eine Methoxygruppe ist;

$R_2$ Wasserstoff, eine Niedrigalkylgruppe, eine Aryl-niedrigalkylgruppe, eine Amminomethylphenyl-niedrigalkylgruppe, eine Hydroxyphenyl-niedrigalkylgruppe, eine Hydroxy-niedrigalkylgruppe, eine Acylaminoniedrigalkylgruppe, eine Amino-niedrigalkylgruppe, eine Dimethylamino-niedrigalkylgruppe, eine Guanidinoniedrigalkylgruppe, eine Imidazolyl-niedrigalkylgruppe, eine Indolyl-niedrigalkylgruppe oder eine Niedrigalkylniedrigthioalkylgruppe;

$R_3$ ein Wasserstoffatom, eine geradkettige oder verzweigte Alkylgruppe mit 1 bis 10 Kohlenstoffatomen, eine Niedrigalkylgruppe, substituiert mit einem oder mehreren Substituenten, ausgewählt aus Halogenatomen, Hydroxylgruppen, Niedrigalkoxygruppen, Aryloxygruppen, substituierten Aryloxygruppen, Heteroaryloxygruppen, substituierten Heteroaryloxygruppen, Aminogruppen, Niedrigalkylaminogruppen, Diniedrigalkylaminogruppen, Acylaminogruppen, Arylaminogruppen, substituierten Arylaminogruppen, Guanidinogruppen, Imidazolylgruppen, Indolylgruppen, Niedrigalkylthiogruppen, Arylthiogruppen, substituierten Arylthiogruppen, Carboxygruppen, Carbamoylgruppen oder Niedrigalkoxycarbonylgruppen, oder $R_3$ eine Arylgruppe, eine substituierte Arylgruppe, eine Niedrigaralkylgruppe, eine Niedrigaralkenylgruppe, eine substituierte Niedrigaralkylgruppe, eine substituierte Niedrigaralkenylgruppe, eine Niedrigheteroaralkylgruppe, eine substituierte Niedrigheteroaralkylgruppe, eine Niedrigheteroaralkenylgruppe, eine substituierte Niedrigheteroaralkenylgruppe, eine Aralkyloxygruppe, eine substituierte Aralkyloxygruppe, eine Heteroaralkyloxygruppe, eine substituierte Heteroaralkyloxygruppe, eine Aralkylthiogruppe, eine substituierte Aralkylthiogruppe, eine Heteroaralkylthiogruppe oder eine substituierte Heteroaralkylthiogruppe, wobei der Arylrest oder der Heteroarylrest der genannten Aryloxygruppen, Heteroaryloxygruppen, Arylaminogruppen, Arylthiogruppen, Arylgruppen, Aralkyloxygruppen, Heteroaralkyloxygruppen, Aralkylthiogruppen, Heteroaralkylthiogruppen, Niedrigaralkylgruppen, Niedrigaralkenylgruppen, Niedrigheteroarlkenylgruppen, substituierten Niedrigheteroaralkylgruppen durch eine oder mehrere Gruppen ausgewählt aus Halogenatomen, Niedrigalkylgruppen, Hydroxygruppen, Niedrigalkoxygruppen, Aminogruppen, Acylaminogruppen, Niedrigalkylaminogruppen, Diniedrigalkylaminogruppen, Carboxylgruppen, Cyanogruppen oder Sulfamoylgruppen substituiert sind; der Alkylrest der genannten Aralkyloxygruppen, Aralkylthiogruppen, Niedrigaralkylgruppen, Niedrigheteroaralkylgruppen, Heteroaralkyloxygruppen und substituierten Heteroalkylthiogruppen durch eine oder mehrere Gruppen substituiert ist ausgewählt aus Halogenatomen, Niedrigalkylgruppen, Hydroxygruppen, Niedrigalkoxygruppen, Aminogruppen, Acylaminogruppen, Niedrigalkylaminogruppen, Diniedrig alkylaminogruppen, Carboxylgruppen, Cyanogruppen oder Sulfamoylgruppen substituiert sind; und

$R_4$ ein Wasserstoffatom oder eine Niedrigalkylgruppe bedeuten.

6. Verwendung der (2S, 3aS, 7aS)-Perhydroindol-2-carbonsäure nach Anspruch 5 zur technischen Herstellung von (2S, 3aS, 7aS)-1-{2-[1-(Ethoxycarbonyl)-(S)-butylamino]-(S)-propionyl}-octahydroindol-2-carbonsäure und deren tert.-Butylaminsalz.

## Claims

### Claims for the following Contracting States : AT BE CH DE FR GB IT LI LU NL SE

1. A process for the industrial preparation of (2S,3aS,7aS)-perhydroindole-2-carboxylic acid of formula (IIIaS) :

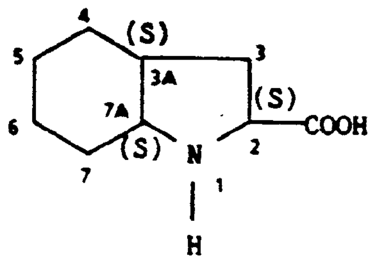

(IIIaS)

characterised in that there is used as starting material an indole-2-carboxylic acid or one of its esters of formula (IV) :

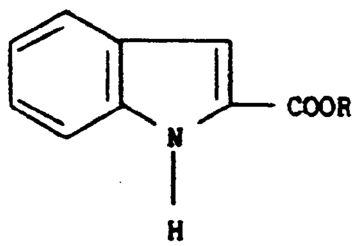

(IV),

optionally converted into a salt by an acid AH,
in which R represents a hydrogen atom or a lower alkyl group,
which is subjected to reduction by a process using the couple tin/hydrochloric acid,
to yield (R,S)-indoline-2-carboxylic acid or one of its esters of formula (V) :

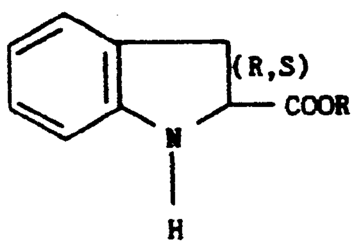

(V)

in which R has the same meaning as in formula (IV), which, when R = H, is the (R,S)-indoline-2-carboxylic acid of formula (VI);
which, when R is other than H, is converted by alkaline hydrolysis into (R,S)-indoline-2-carboxylic acid of formula (VI) :

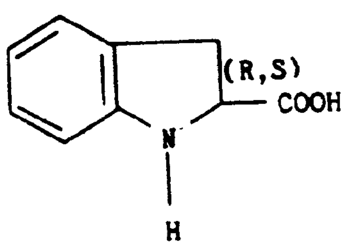

(VI)

which, in fact, comprises a mixture of two isomers according to how the carboxy-carrying carbon atom is configured :

– in the R configuration (R isomer),

– in the S configuration (S isomer),

a mixture from which the S isomer is isolated by adding the said mixture to a solution of (+)-α-methylbenzylamine in a lower aliphatic alcohol, to obtain

a precipitate of the salt of (S)-indoline-2-carboxylic acid with (+)-α-methylbenzylamine,

which, after filtration, is dissolved in water, the solution obtained then being acidified to allow the (S)-indoline-2-carboxylic acid to be liberated,

which acid is subjected to catalytic hydrogenation, the catalyst being selected from nickel, platinum, palladium and rhodium in admixture with a support such as carbon, so as to obtain (2s, 3as, 7as)-perhydroindole-2-carboxylic acid, the latter being separated from the small proportion of (2S, 3aR, 7aR)-isomer by a single crystallisation in a polar solvent carefully selected from a lower aliphatic alcohol, acetonitrile, dioxan and ethyl acetate, alone, or mixed with one another or with water provided the mixture is monophasic.

2. A process for the industrial synthesis of (2S, 3aS, 7aS)-perhydroindole-2-carboxylic acid according to claim 1, characterised in that there is used as starting material the ethyl ester of indole-2-carboxylic acid of formula (IVo) :

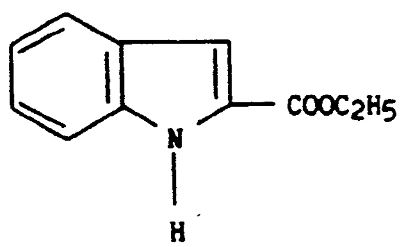

(IVo)

which is itself obtained by esterifying indole-2-carboxylic acid with ethanol in the presence of an esterification catalyst, such as sulphuric acid.

3. A process according to either claim 1 or claim 2, characterised in that the reduction of the (S)-indoline-2carboxylic acid to perhydroindole-2-carboxylic acid is carried out using rhodium-on-carbon as catalyst.

4. A process according to any one of claims 1 to 3, characterised in that the recrystallisation for the purpose of isolating (2S, 3aS, 7aS)-perhydroindole-2-carboxylic acid from the reaction mixture obtained after the catalytic reduction of (S)-indoline-2-carboxylic acid is carried out using a dioxan/water mixture as solvent.

5. The use of the (2S, 3aS, 7aS)-perhydroindole-2-carboxylic acid obtained according to any one of claims 1 to 4 for the preparation of carboxyalkyl dipeptides of formula (I)

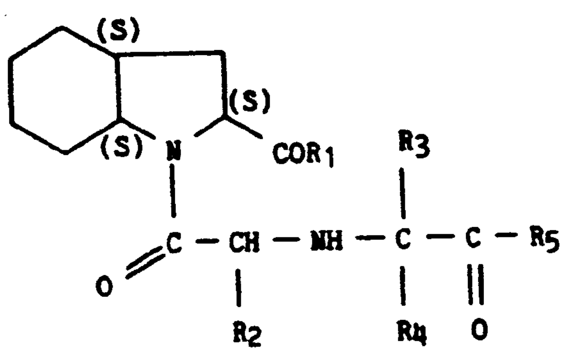

(I)

and their pharmaceutically acceptable salts, in which:

$R_1$ and $R_5$, which are the same or different, represent hydroxy, lower alkoxy, lower alkenyloxy, di-lower alkylamino-lower alkoxy, acylamino-lower alkoxy, acyloxy-lower alkoxy, aryloxy, aryl-lower alkoxy, amino, lower alkylamino, di-lower alkylamino, hydroxyamino, aryl-lower alkylamino or substituted aryloxy or substituted aryl-lower alkoxy wherein the substituent is methyl, halo or methoxy;

$R_2$ is hydrogen, lower alkyl, aryl-lower alkyl, amino-methylphenyl-lower alkyl, hydroxyphenyl-lower alkyl, hydroxy-lower alkyl, acylamino-lower alkyl, amino-lower alkyl, dimethylamino-lower alkyl, guanidino-lower alkyl, imidazolyl-lower alkyl, indolyl-lower alkyl or lower alkylthio-lower alkyl;

$R_3$ is hydrogen, a straight-chain or branched alkyl having from 1 to 10 carbon atoms, a lower alkyl substituted by one or more substituents selected from halo, hydroxy, lower alkoxy, aryloxy, substituted aryloxy,

heteroaryloxy, substituted heteroaryloxy, amino, lower alkylamino, di-lower alkylamino, acylamino, arylamino, substituted arylamino, guanidino, imidazolyl, indolyl, lower alkylthio, arylthio, substituted arylthio, carboxy, carbamoyl and lower alkoxycarbonyl, or $R_3$ is aryl, substituted aryl, aryl-lower alkyl, aryl-lower alkenyl, substituted aryl-lower alkyl, substituted aryl-lower alkenyl, heteroaryl-lower alkyl, substituted heteroaryl-lower alkyl, heteroaryl-lower alkenyl, substituted heteroaryl-lower alkenyl, aralkoxy, substituted aralkoxy, heteroaralkoxy, substituted heteroaralkoxy, aralkylthio, substituted aralkylthio, heteroaralkylthio or substituted heteroaralkylthio, the aryl or heteroaryl moiety of the above substituted aryloxy, heteroaryloxy, arylamino, arylthio, aryl, aralkoxy, heteroaralkoxy, aralkylthio, heteroaralkylthio, aryl-lower alkyl, aryl-lower alkenyl, heteroaryl-lower alkenyl and heteroaryl-lower alkyl being substituted by one or more groups selected from halo, lower alkyl, hydroxy, lower alkoxy, amino, acylamino, lower alkylamino, di-lower alkylamino, carboxy, cyano and sulphamoyl; the alkyl moiety of the said substituted aralkoxy, aralkylthio, aryl-lower alkyl, heteroaryl-lower alkyl, heteroaralkoxy and heteroaralkylthio being substituted by one or more groups also selected from halo, lower alkyl, hydroxy, lower alkoxy, amino, acylamino, lower alkylamino, di-lower alkylamino, carboxy, cyano and sulphamoyl; and

$R_4$ is hydrogen or a lower alkyl group.

6. The use according to claim 5 of (2aS, 3aS, 7aS)-perhydroindole-2-carboxylic acid for the industrial preparation of (2S, 3aS, 7aS)-1-{2(S)-[1(S)-(ethoxycarbonyl)butylamino]propionyl}octahydroindole-2-carboxylic acid from its tert.-butylamine salt.

**Claims for the following Contracting States : ES GR**

1. A process for the industrial preparation of (2S,3aS,7aS)-perhydroindole-2-carboxylic acid of formula (IIIas) :

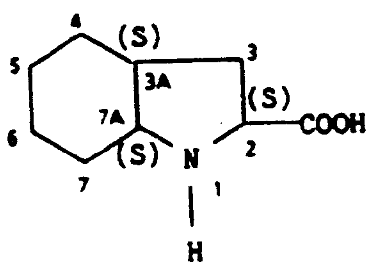

(IIIaS)

characterised in that there is used as starting material an indole-2-carboxylic acid or one of its esters of formula (IV) :

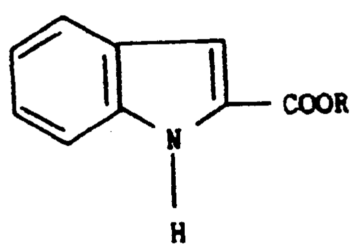

(IV),

optionally converted into a salt by an acid AH,
in which R represents a hydrogen atom or a lower alkyl group,
which is subjected to reduction by a process using the couple tin/hydrochloric acid,
to yield (R,S)-indoline-2-carboxylic acid or one of its esters of formula (V) :

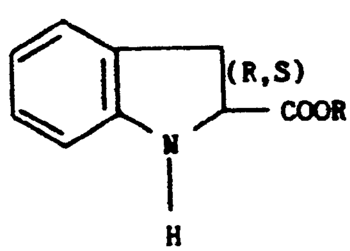

(V)

in which R has the same meaning as in formula (IV), which, when R = H, is the (R,S)-indoline-2-carboxylic acid of formula (VI);
which, when R is other than H, is converted by alkaline hydrolysis into (R,S)-indoline-2-carboxylic acid of formula (VI) :

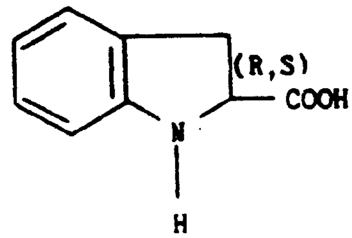

(VI)

which, in fact, comprises a mixture of two isomers according to how the carboxy-carrying carbon atom is configured :

- in the R configuration (R isomer),
- in the S configuration (S isomer),

a mixture from which the S isomer is isolated by adding the said mixture to a solution of (+)-α-methylbenzylamine in a lower aliphatic alcohol, to obtain
a precipitate of the salt of (S)-indoline-2-carboxylic acid with (+)-α-methylbenzylamine,
which, after filtration, is dissolved in water, the solution obtained then being acidified to allow the (S)-indoline-2-carboxylic acid to be liberated,
which acid is subjected to catalytic hydrogenation, the catalyst being selected from nickel, platinum, palladium and rhodium in admixture with a support such as carbon, so as to obtain (2S, 3aS, 7aS)-perhydroindole-2-carboxylic acid, the latter being separated from the small proportion of (2S, 3aR, 7aR)-isomer by a single crystallisation in a polar solvent carefully selected from a lower aliphatic alcohol, acetonitrile, dioxan and ethyl acetate, alone, or mixed with one another or with water provided the mixture is monophasic.

2. A process for the industrial synthesis of (2S, 3aS, 7aS)-perhydroindole-2-carboxylic acid according to claim 1, characterised in that there is used as starting material the ethyl ester of indole-2-carboxylic acid of formula (IVo) :

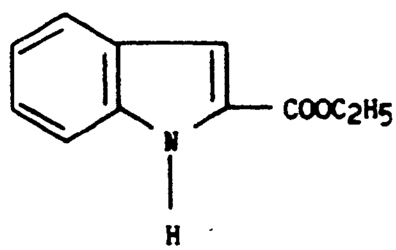

(IVo)

which is itself obtained by esterifying indole-2-carboxylic acid with ethanol in the presence of an esterification catalyst, such as sulphuric acid.

3. A process according to either claim 1 or claim 2, characterised in that the reduction of the (S)-indoline-2carboxylic acid to perhydroindole-2-carboxylic acid is carried out using rhodium-on-carbon as catalyst.

4. A process according to any one of claim 1 to 3, characterised in that the recrystallisation for the purpose of isolating (2S, 3aS, 7aS)-perhydroindole-2-carboxylic acid from the reaction mixture obtained after the catalytic reduction of (S)-indoline-2-carboxylic acid is carried out using a dioxan/water mixture as solvent.

5. The use of the (2S, 3aS, 7aS)-perhydroindole-2-carboxylic acid obtained according to any one of claims 1 to 4 for the preparation of carboxyalkyl dipeptides of formula (I) :

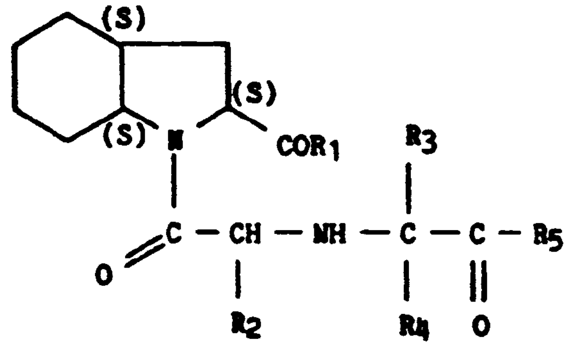

(I)

and their pharmaceutically acceptable salts, in which:

$R_1$ and $R_5$, which are the same or different, represent hydroxy, lower alkoxy, lower alkenyloxy, di-lower alkylamino-lower alkoxy, acylamino-lower alkoxy, acyloxy-lower alkoxy, aryloxy, aryl-lower alkoxy, amino, lower alkylamino, di-lower alkylamino, hydroxyamino, aryl-lower alkylamino or substituted aryloxy or substituted aryl-lower alkoxy wherein the substituent is methyl, halo or methoxy;

$R_2$ is hydrogen, lower alkyl, aryl-lower alkyl, amino-methylphenyl-lower alkyl, hydroxyhenyl-lower alkyl, hydroxy-lower alkyl, acylamino-lower alkyl, amino-lower alkyl, dimethylamino-lower alkyl, guanidino-lower alkyl, imidazolyl-lower alkyl, indolyl-lower alkyl or lower alkylthio-lower alkyl;

$R_3$ is hydrogen, a straight-chain or branched alkyl having from 1 to 10 carbon atoms, a lower alkyl substituted by one or more substituents selected from halo, hydroxy, lower alkoxy, aryloxy, substituted aryloxy, heteroaryloxy, substituted heteroaryloxy, amino, lower alkylamino, di-lower alkylamino, acylamino, arylamino, substituted arylamino, guanidino, imidazolyl, indolyl, lower alkyl-thio, arylthio, subtituted arylthio, carboxy, carbamoyl and lower alkoxycarbonyl, or $R_3$ is aryl, substituted aryl, aryl-lower alkyl, aryl-lower alkenyl, substituted aryl-lower alkyl, substituted aryl-lower alkenyl, heteroaryl-lower alkyl, substituted heteroaryl-lower alkyl, heteroaryl-lower alkenyl, substituted heteroaryl-lower alkenyl, aralkoxy, substituted aralkoxy, heteroaralkoxy, substituted heteroaralkoxy, aralkylthio, substituted aralkylthio, heteroaralkylthio or substituted heteroaralkylthio, the aryl or heteroaryl moiety of the above substituted aryloxy, heteroaryloxy, arylamino, arylthio, aryl, aralkoxy, heteroaralkoxy, aralkylthio, heteroaralkylthio, aryl-lower alkyl, aryl-lower alkenyl, heteroaryl-lower alkenyl and heteroaryl-lower alkyl being subtituted by one or more groups selected from halo, lower alkyl, hydroxy, lower alkoxy, amino, acylamino, lower alkylamino, di-lower alkylamino, carboxy, cyano and sulphamoyl; the alkyl moiety of the said substituted aralkoxy, aralkylthio, aryl-lower alkyl, heteroaryl-lower alkyl, heteroaralkoxy and heteroaralkylthio being substituted by one or more groups also selected from halo, lower alkyl, hydroxy, lower alkoxy, amino, acylamino, lower alkylamino, di-lower alkylamino, carboxy, cyano and sulphamoyl; and

$R_4$ is hydrogen or a lower alkyl group.

6. The use according to claim 5 of (2aS, 3aS, 7aS)-perhydroindole-2-carboxylic acid for the industrial preparation of (2S, 3aS, 7aS)-1-{2(S)-[1(S)-(ethoxycarbonyl)butylamino]propionyl}octahydroindole-2-carboxylic acid from its tert.-butylamine salt.